(19) Europäisches Patentamt
European Patent Office
Office européen des brevets

(11) Publication number: **0 353 959**
**A2**

(12) **EUROPEAN PATENT APPLICATION**

(21) Application number: 89307693.5

(22) Date of filing: 28.07.89

(51) Int. Cl.4 C07H 19/01 , C07D 493/22 , A61K 31/70 , A61K 31/365 , A01N 43/90 , //(C07D493/22, 313:00,311:00,311:00,307:00)

Claim for the following Contracting State: ES.

The title of the invention has been amended (Guidelines for Examination in the EPO. A-III, 7.3).

(30) Priority: 02.08.88 GB 8818373
21.10.88 GB 8824761
01.11.88 GB 8825562
03.05.89 GB 8910089

(43) Date of publication of application:
07.02.90 Bulletin 90/06

(84) Designated Contracting States:
AT BE CH DE ES FR GB GR IT LI LU NL SE

(71) Applicant: BEECHAM GROUP PLC
Beecham House Great West Road
Brentford Middlesex TW8 9BD(GB)

(72) Inventor: Baker, Geoffrey Harold Beecham Pharmaceuticals
Great Burgh Yew Tree Bottom Road
Epsom Surrey KT18 5XO(GB)

Inventor: Dorgan, Roderick John Beecham Pharmaceuticals
Great Burgh Yew Tree Bottom Road
Epsom Surrey KT18 5XQ(GB)

Inventor: Morgan, David Owen Beecham Pharmaceuticals
Great Burgh Yew Tree Bottom Road
Epsom Surrey KT18 5XQ(GB)

Inventor: Banks, Rhona Mary Beecham Pharmaceuticals
Walton Oaks Dorking Road
Tadworth Surrey KT20 7NT(GB)

Inventor: Blanchflower, Simon Edward Beecham Pharmaceuticals
Walton Oaks Dorking Road
Tadworth Surrey KT20 7NT(GB)

Inventor: Shelley, Peter Robin
Beecham Pharmaceuticals Brockham Park
Betchworth Surrey RH3 7AJ(GB)

(74) Representative: West, Vivien et al
Beecham Pharmaceuticals Great Burgh Yew Tree Bottom Road
Epsom Surrey KT18 5XQ(GB)

(54) **Antihelmintic macrolide antibiotics.**

(57) There are disclosed novel compounds of formula (I)

EP 0 353 959 A2

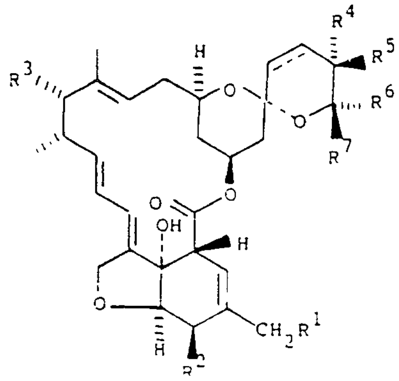

(I)

wherein $R^1$ is hydrogen or optionally protected hydroxy; $R^2$ is alkoxy, optionally protected hydroxy, oxo or optionally O-substituted oximino; $R^3$ is hydrogen, optionally protected hydroxy, or a group 4′-(α-L-oleandrosyl)-α-L-oleandrosyloxy or α-L-oleandrosyloxy wherein the terminal hydroxy group is optionally protected; the dashed line represents an optional double bond; and $R^4$, $R^5$, $R^6$ and $R^7$ are the same or different and each is hydrogen or an organic radical; as well as a process for their production and their use as endectocides and pesticides.

## NOVEL COMPOUNDS

The present invention relates to novel anthelmintic compounds, to processes for their production, to pharmaceutical formulations containing them, and to their use in human or veterinary medicine.

The milbemycins and avermectins are a group of macrolide antibiotics which have been prepared by the cultivation of microorganisms and are described in inter alia GB-A-1,390,336, J. Antibiotics 29(3), 76-14 to 76-16 and 29 (6), 76-35 to 76-42, GB-A-2 170 499, EP-A-O 073 660 and EP-A-0 204 421. They have anthelmintic activity. Further anthelmintically active milbemycins and avermectins are described in GB-A-2 176 180, EP-A-0 212 867, EP-A-0 237 339, EP-A-0 241 146, EP-A-0 214 731, EP-A-0 194 125, EP-A-0 170,006, and US-A-4,285,963.

The compounds disclosed in the above references include compounds of formula (A):

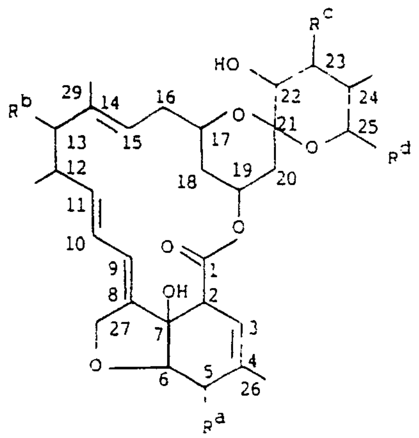

(A)

wherein $R^a$ is methoxy or hydroxy, $R^b$ is hydrogen, $R^c$ is hydrogen, pentanoyloxy, heptanoyloxy, or 2-methylhexanoyloxy, and $R^d$ is methyl or ethyl, with the proviso that when $R^c$ is hydrogen, $R^a$ is methoxy; or $R^a$ is methoxy or hydroxy, $R^b$ is hydrogen, $R^c$ is 2-methylbutanoyloxy, 2,4-dimethylpent-2-enoyloxy, or 2,4-dimethylpentanoyloxy, and $R^d$ is methyl or ethyl, with the proviso that when $R^d$ is ethyl, $R^a$ is hydroxy and $R^c$ is 2,4-dimethylpentanoyloxy; or $R^a$ is methoxy or hydroxy, $R^b$ is the group of formula:

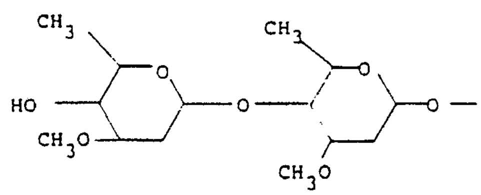

(4′-(α-L-oleandrosyl)-α-L-oleandrosyloxy), $R^c$ is hydroxy, and $R^d$ is 1-methyl propyl.

EP-A-O 254 583 (USSN 076,274) described compounds of formula (B):

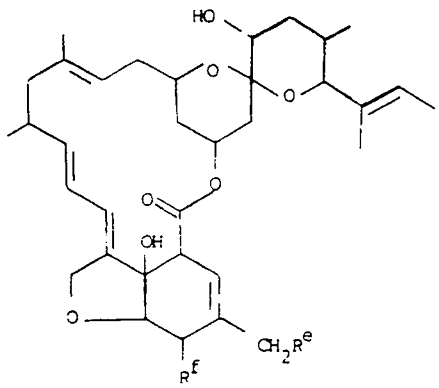

(B)

wherein $R^e$ is hydrogen or E 2-methyl 2-butenoyloxy, and $R^f$ is methoxy or hydroxy, with the proviso that when $R^e$ is E 2-methyl 2-butenoyloxy, $R^f$ is methoxy.

EP-A-0 325 462 (USSN 299,933) describes compounds of formula (C):

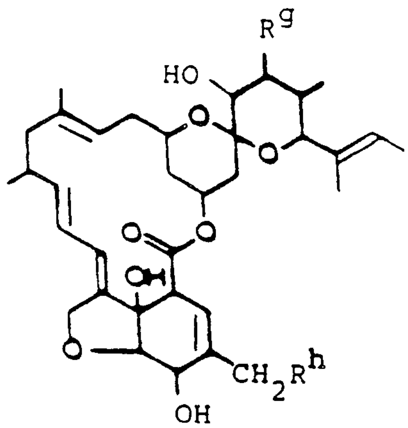

(C)

wherein $R^g$ and $R^h$ are as set out in the following table:

| Compound | $R^g$ | $R^h$ |
|---|---|---|
| VM48130 | $O-CO-CH(CH_3)_2$ | H |
| VM48633 | H | $O-CO-CH=C(CH_3)_2$ |
| VM47704 | H | $O-CO-CH_2-CH(CH_3)_2$ |
| VM48642 | H | $O-CO-CH_2$ 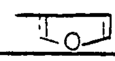 |

EP-A-0 288 205 (USSN 183,581) discloses compounds of formula (D):

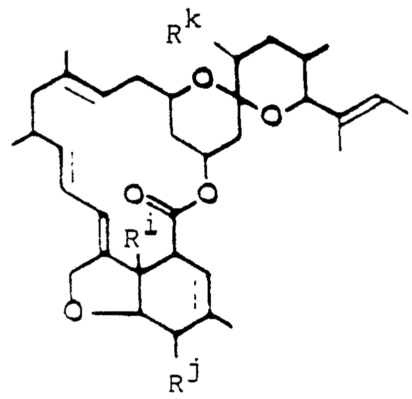

(D)

wherein $R^i$ is hydroxy or methoxy and $R^j$ and $R^k$ are the same or different and each is selected from optionally protected hydroxy, alkoxy, acyloxy, sulphonyloxy, oxo and optionally O-substituted oximino.

The absolute configuration of the compounds of formulae (A) to (D) is believed to be as follows:

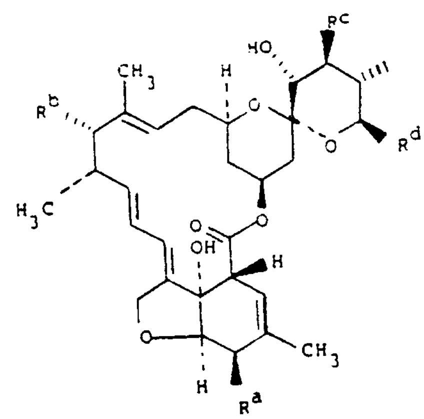

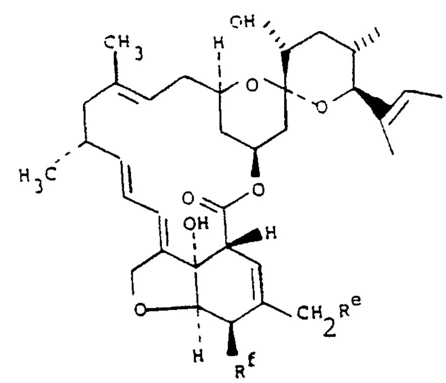

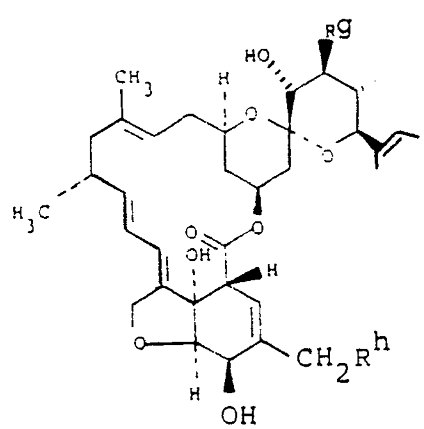

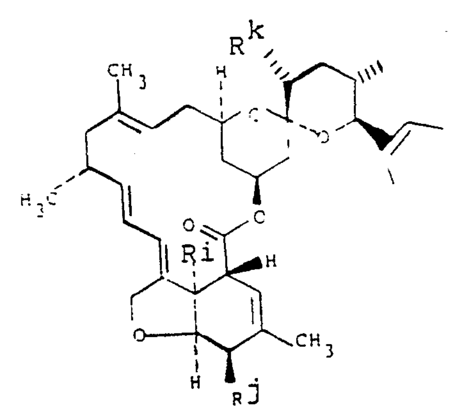

The following publications describe avermectin and milbemycin derivatives which are unsubstituted at positions 22 and 23 and are substituted by an organic radical at positions 24 and/or 25. These compounds are natural products or are obtained from natural products <u>via</u> specific processes:

GB-A-1,573,955
GB-A-1,390,336
GB-A-2 176 182
EP-A-0 212 867
EP-A-0 237 339
EP-A-0 241 146
EP-A-0 214 731
EP-A-0 194 125

EP-A-0 252 879
EP-A-0 246 739
EP-A-0 298 423
EP-A-0 317 148
EP-A-0 316 124

We have now discovered that it is possible to prepare novel compounds from starting materials having a hydroxy substituent at the C-22 position, and that these compounds are useful as anthelmintically active compounds.

According to the present invention there is provided a compound of formula (I):

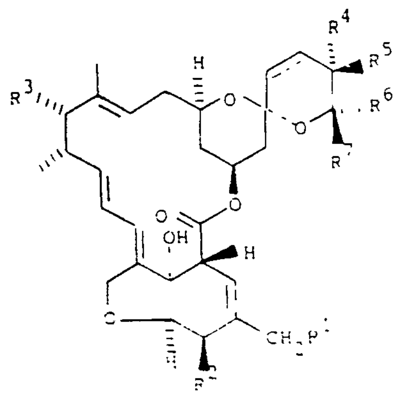

(I)

wherein $R^1$ is hydrogen or optionally protected hydroxy; $R^2$ is alkoxy, optionally protected hydroxy, oxo or optionally O-substituted oximino; $R^3$ is hydrogen, optionally protected hydroxy, or a group 4'-(α-L-oleandrosyl)-α-L-oleandrosyloxy or α-L-oleandrosyloxy wherein the terminal hydroxy group is optionally protected; the dashed line represents an optional double bond; and $R^4$, $R^5$, $R^6$ and $R^7$ are the same or different and each is hydrogen or an organic radical; with the proviso that the compound of formula (I) is not a compound disclosed by GB-A-1,573,955, GB-A-1,390,336, GB-A-2 176 182, EP-A-0 212 867, EP-A-0 237 339, EP-A-0 241 146, EP-A-0 214 731, EP-A-0 194 125, EP-A-0 246 739, EP-A-0 316 124 or EP-A-0 317 148.

Preferably, (a) when $R^1$ is optionally protected hydroxy, $R^3$ is hydrogen, and/or (b) when $R^4$ is methyl and $R^5$ and $R^6$ are hydrogen, $R^7$ does not have any of the values set out in Table I hereinbelow, and/or (c) when the double bond is absent, $R^4$ is methyl and $R^5$ and $R^6$ are hydrogen, $R^7$ does not have any of the values set out in Table II hereinbelow, and/or (d) when the double bond is absent, $R^4$ is hydroxymethyl and $R^5$ and $R^6$ are hydrogen, $R^7$ is not sec butyl, and/or (e) when $R^2$ is not methoxy or optionally protected hydroxy, $R^1$ and $R^3$ are both hydrogen.

TABLE I

$-CH(CH_3)_2$, $-CH(CH_3)C_2H_5$

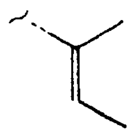
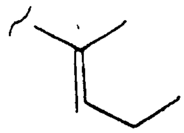
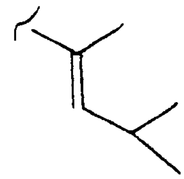

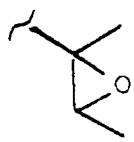
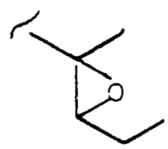
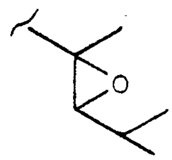

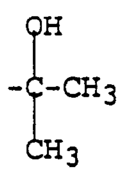
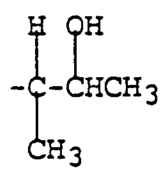

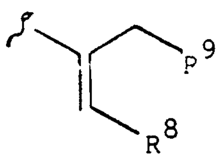

$R^8$ is methyl, ethyl or isopropyl

$R^9$ is $C_{1-7}$ alkyl, phenyl or $C_{7-14}$ phenalkyl

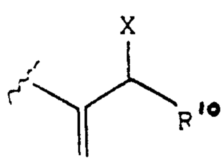

X is halogen

$R^{10}$ is methyl, ethyl or isopropyl.

1-methylthioethyl, cyclobutyl, cyclopentyl, cyclohexyl, 2-methylcyclopropyl, cyclohex-3-enyl, or thien-3-yl.

TABLE II

| | |
|---|---|
| $-CH_3$ | 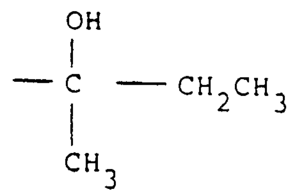 |
| $-C_2H_5$ | 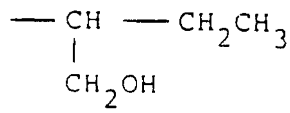 |

Preferably, when $R^4$ is methyl and $R^5$ and $R^6$ are hydrogen, $R^7$ is not a group:

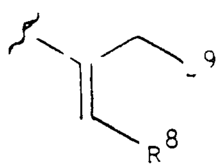

wherein $R^8$ is methyl, ethyl or isopropyl and $R^9$ is alkyl, phenalkyl or phenyl.

It is also preferred that, when $R^4$ is methyl and $R^5$ and $R^6$ are hydrogen, $R^7$ is not selected from the group consisting of pent-2-yl, 3-methylbut-2-yl, hex-2-yl, pent-4-en-2-yl, 1-cyclopropylethyl, cycloheptyl, 4,4-difluorocyclohexyl, 4-methylenecyclohexyl, 3-methylcyclohexyl, cyclopenten-1-yl, cyclohexen-1-yl, tetrahydropyran-4-yl, thien-2-yl, 3-furyl, and 2-chlorothien-4-yl.

In a particular aspect, when $R^4$ is methyl and $R^5$ and $R^6$ are hydrogen, $R^7$ is not an alpha-branched $C_{3-8}$ alkylthioalkyl group; a $C_{3-8}$ cycloalkyl group optionally substituted by one $C_{1-4}$ alkyl group; a $C_{5-8}$ cycloalkenyl group; or a 3 to 6 membered sulphur containing heterocyclic ring. More especially, $R^7$ is not an alpha-branched $C_{3-8}$ alkyl, alkenyl or alkylthioalkyl group; a $C_{5-8}$ cycloalkylalkyl group wherein the alkyl group is an alpha-branched $C_{2-5}$ alkyl group; a $C_{3-8}$ cycloalkyl group optionally substituted by methylene or one or more $C_{1-4}$ alkyl groups or halo atoms; a $C_{5-8}$ cycloalkenyl group; or a 3 to 6 membered oxygen or sulphur containing heterocyclic ring which may optionally be substituted by one or more halo atoms.

In a still further particular aspect, when $R^4$ is methyl and $R^5$ and $R^6$ are hydrogen, $R^7$ is not an alpha-branched $C_3$-$C_8$ alkyl,alkenyl, alkynyl, alkoxyalkyl or alkylthioalkyl group; a $C_5$-$C_8$ cycloalkylalkyl group wherein the alkyl group is an alpha-branched $C_{2-5}$ alkyl group; a $C_3$-$C_8$ cycloalkyl or $C_{5-8}$ cycloalkenyl group, either of which may optionally be substituted by methylene or one or more $C_1$-$C_4$ alkyl groups or halo atoms; or a 3 to 6 membered oxygen or sulphur containing heterocyclic ring which may be saturated, or fully or partially unsaturated and which may optionally be substituted by one or more $C_1$-$C_4$ alkyl groups or halo atoms. More especially, $R^7$ is not an alpha-branched alkyl, alkenyl, alkynyl, alkoxyalkyl or alkylthioalkyl group; a cycloalkylalkyl group wherein the alkyl group is alpha-branched; a cycloalkyl or cycloalkenyl group, either of which may optionally be substituted by methylene or one or more alkyl groups or halo atoms; or an oxygen or sulphur containing heterocyclic ring which may be saturated, or fully or partially unsaturated and which may optionally be substituted by one or more alkyl groups or halo atoms.

According to a further preferred aspect, when $R^4$ is ethyl and $R^5$ and $R^6$ are hydrogen, $R^7$ is not (Z)-4-methyl-pent-2-en-2-yl.

According to a still further preferred aspect, when $R^4$ is methyl and $R^5$ is hydrogen, the 25-position is not substituted in the same way as that of compound III of US-A-4,285,963.

Suitable protecting groups for hydroxy include TBDMS (t-butyldimethylsilyl), and acyl. Further suitable protecting groups are described in, for example, "Protective Groups in Organic Synthesis" Theodora W. Greene, Wiley-Interscience 1981 Ch 2, 10-86.

When any of $R^4$ to $R^7$ is an organic radical it may advantageously be selected from the group consisting of alkyl, alkenyl, alkynyl, aryl, heterocyclyl, mono-, bi-or tri- cycloalkyl, cycloalkenyl and aralkyl.

As used herein alkyl includes straight and branched $C_{1-20}$, more especially $C_{1-12}$, particularly $C_{1-6}$ alkyl, and alkenyl and alkynyl include straight and branched $C_{2-20}$, more especially $C_{2-12}$, particularly $C_{2-6}$ alkenyl and alkynyl.

When any of $R^4$ to $R^7$ comprises an alkyl, alkenyl or alkynyl moiety that moiety may optionally be substituted by one or more substituents selected from the group consisting of hydroxy, oxo, alkoxy, alkylthio, halogen and trifluoromethyl.

When used herein the term 'aryl' includes phenyl and naphthyl optionally substituted with up to five, preferably up to three, groups selected from halogen, $C_{1-6}$ alkyl, phenyl, $C_{1-6}$ alkoxy, halo substituted ($C_{1-6}$) alkyl, hydroxy, amino, nitro, carboxy, $C_{1-6}$ alkoxycarbonyl, $C_{1-6}$ alkoxycarbonyl-($C_{1-6}$)-alkyl, $C_{1-6}$ alkylcarbonyloxy, or $C_{1-6}$ alkylcarbonyl groups.

The term 'heterocyclyl' includes saturated, unsaturated and aromatic single or fused rings comprising up to four hetero atoms in the ring selected from oxygen, nitrogen and sulphur and optionally substituted with up to three halogen, $C_{1-6}$ alkyl, $C_{1-6}$ alkoxy, halo-($C_{1-6}$)-alkyl, hydroxy, amino, carboxy, $C_{1-6}$ alkoxycarbonyl, $C_{1-6}$ alkoxycarbonyl($C_{1-6}$) alkyl, aryl or oxo groups.

Suitably the heterocyclic ring comprises from 4 to 7 ring atoms, preferably 5 to 6 atoms.

The term 'halogen' refers to fluorine, chlorine, bromine and iodine.

Suitable substituents for an oxime are as set out in EP-A-0 288 205.

As used herein cycloalkyl includes $C_{3-12}$, more especially $C_{4-8}$, cycloalkyl, $C_{6-12}$, more especially $C_{7-10}$, bicycloalkyl, and $C_{7-14}$, more especially $C_{9-12}$, tricycloalkyl, and cycloalkenyl includes $C_{4-12}$, more especially $C_{5-8}$ cycloalkenyl. When any of $R_4$ to $R_7$ comprises a cycloalkyl or cycloalkenyl moiety, that moiety may be substituted as set out above for alkyl, alkenyl, and alkynyl, and/or by one or more substituents selected from the group consisting of methylene and alkyl.

Any two of $R^4$ to $R^7$ may be taken together with the carbon atom(s) to which they are attached to designate a cycloalkyl, cycloalkenyl, aryl or heterocyclyl group which may optionally be substituted as set out above.

In a preferred aspect of the invention, $R^1$ is hydrogen, $R^2$ is methoxy or hydroxy, and $R^3$ is hydrogen.

In a particular aspect of the invention, $R^5$ and $R^6$ are hydrogen, one of $R^4$ and $R^7$ is hydrogen or $C_{1-3}$ alkyl, more especially methyl, and the other of $R^4$ and $R^7$ is an organic radical, more especially an aryl, cycloalkyl or alkyl group.

Preferably, $R^2$ is a hydroxy group.

The compound or mixture of compounds according to the invention is suitably provided in substantially pure form, for example at least 50% pure, suitably at least 60% pure, advantageously at least 75% pure, preferably at least 85% pure, more preferably at least 95% pure, especially at least 98% pure, all percentages being calculated as weight/weight. An impure or less pure form of a compound according to the invention may, for example, be used in the preparation of a more pure form of the same compound or of a related compound (for example a corresponding derivative) suitable for pharmaceutical use.

The compounds of the invention have parasiticidal properties, for example against nematodes such as Trichostrongylus colubriformis, and are useful for the treatment of helminthiasis in animals such as mammals, including humans and domesticated animals (including farm animals).

Accordingly the present invention also provides a compound according to the invention, for use in the treatment of the human or animal body, especially for treating endo- and ectoparasitic infestations and particularly for treating helminthiasis of domestic and farm animals.

The term helminthiasis encompasses those diseases of man and animals caused by infestation with parasitic worms such as Strongyles, Ascarids, hookworms lungworms, filarial worms and whipworms. The compound may also be used against nematodes occurring in the soil or parasitic to plants.

The compounds of the invention are also active against Arthropods. The phylum Arthropoda comprises insects -such as biting flies, lice, bugs, beetles and fleas -and arachnids - such as mites and ticks.

Thus, a broad aspect of the invention provides a method of eradicating arthropod or nematode infestations, which method comprises applying a compound according to the invention or a derivative thereof to the arthropods or nematodes or to their environment.

The present invention thus provides a pesticidal composition comprising a compound according to the invention or a derivative thereof together with a suitable carrier or excipient, such as an aerosol formulation.

The present invention also provides a pharmaceutical or veterinary composition comprising a compound according to the invention or a pharmaceutically acceptable derivative thereof together with a pharmaceutically or veterinarily acceptable carrier or excipient.

The present invention also provides a method of treatment or prophylaxis of endo- and ectoparasitic infestations, especially helminthiasis, of animals, especially humans and domesticated mammals, which comprises administering an effective non-toxic amount of a compound according to the invention or a pharmaceutically acceptable derivative thereof, or a composition according to the invention, to a patient in need thereof.

The composition according to the invention may be formulated for administration in any convenient way for use in human or veterinary medicine, by analogy with other anthelmintics.

In suitable formulations the drug may be administered to animals orally (as a paste, drench, bolus, capsule or tablet), parenterally, percutaneously, as a food additive (eg granules, pellets or powder), or may be prepared as an aerosol spray formulation.

The compounds of the invention may be formulated as a mixture with each other and/or with other anthelmintics, insecticides, acaricides or other pharmacologically active substances.

Suitably the composition consists of sufficient material to provide a dose of from 0.001 to 100mg of active ingredient per kg of animal body weight per dose, more suitably 0.01 to 10mg/kg per dose.

A composition according to the invention may suitably contain from 0.1% by weight, preferably from 1.0 to 60% by weight, of the compound according to the invention (based on the total weight of the composition), depending on the method of administration.

It will be appreciated that, in some cases, it will be advisable to repeat the dosing of the infected or potentially infected human or animal with the compound of the invention according to conventional dosage regimes used with anthelmintics.

A further aspect of the invention provides a process for the preparation of a compound of formula (I), which process comprises the cyclisation of a compound of formula (II), for example in the presence of an acid catalyst:

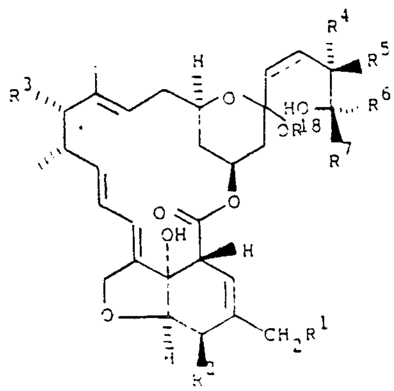

(II)

wherein $R^1$ to $R^7$ and the dashed line have the values set out above, and $R^{18}$ is hydrogen or lower alkyl, more particularly $C_{1-3}$ alkyl; and/or, optionally, the conversion of a compound of formula (I) wherein the dashed line represents a double bond to a compound of formula (I) wherein the dashed line represents a single bond, by hydrogenation in the presence of a suitable catalyst, such as Wilkinsons catalyst.

It should be appreciated that the configuration at C21 of the compound of formula (I) will be subject to thermodynamic control (as discussed eg by P. Deslongchamps et al, Canadian J. Chem. [1981] 59, 1105): it is believed that the epimer in which the configuration at C21 is as in the naturally occurring compounds of formulae (A), (B) and (C) is normally thermodynamically favoured.

Compounds of formula (II) may be obtained from compounds of formula (III):

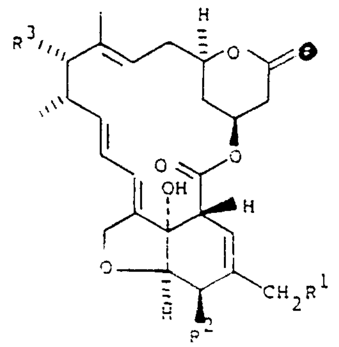

(III)

wherein R' to $R^3$ are as defined above, via the routes shown in Scheme I below:

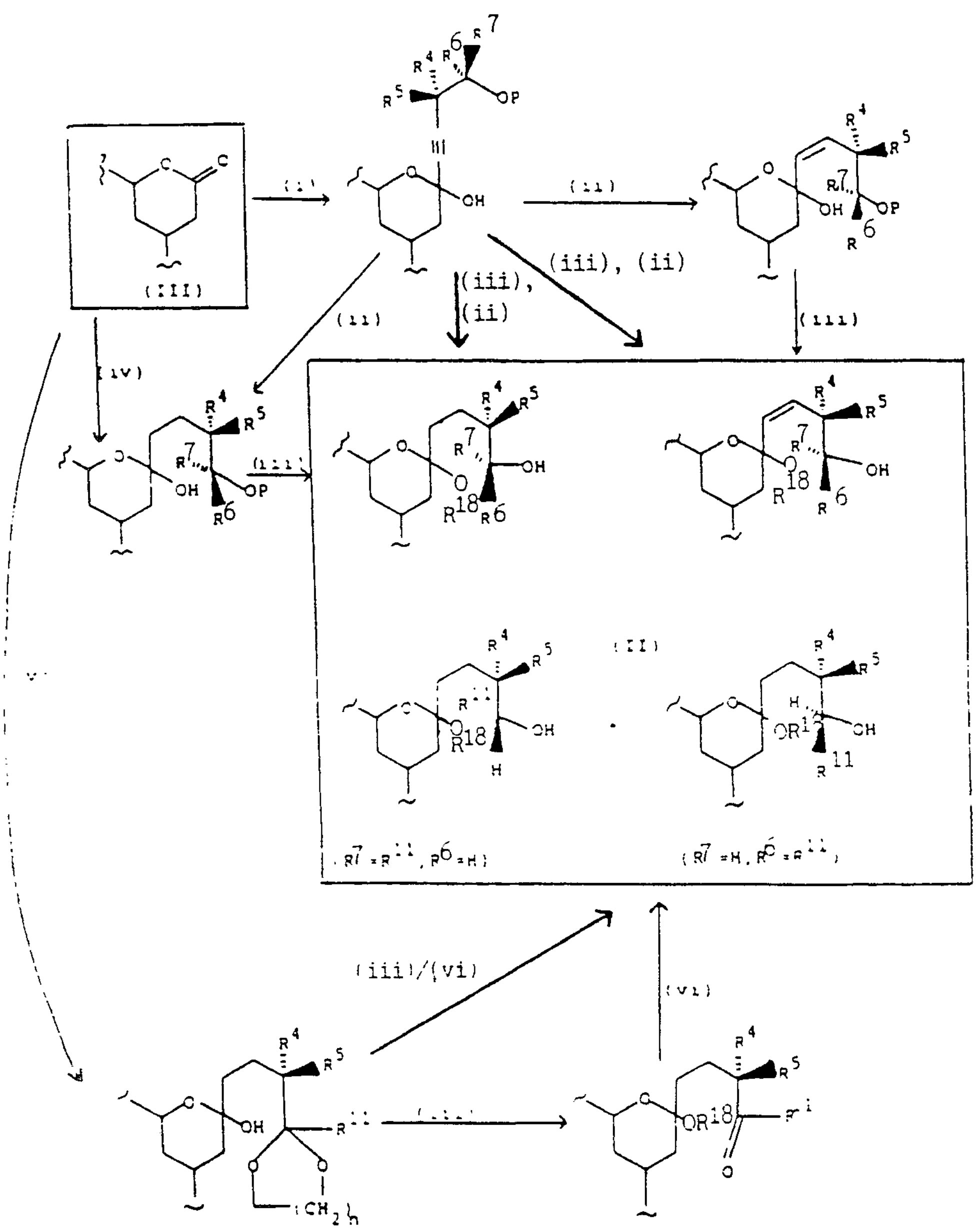


Notes

(i) $MC{\equiv}C.CR^4R^5.CR^6R^7OP$ where M is a metalating agent such as Li or BrMg and P is an acid labile protecting group such as THP (tetrahydropyranyl)

(ii) $H_2$/Lindlar catalyst or other suitable catalyst

(iii) $R^{18}OH/H+$

(iv) $XMg(CH_2)_2CR^4R^5CR^6R^7OP$ where X is halogen

(v) $XMg(CH_2)_2CR^4R^5CR^{11}<^{O-(CH_2)_n}_{O}$ where $R^{11}$ is hydrogen or an organic radical and n=1 or 2

(vi) $NaCNBH_3$

Compounds of formula (III) can be prepared as described in EP-A-0,319,142 (USSN 265,509) by cleaving the 21-22 carbon-carbon bond of a precursor having a hydroxy substituted on C22, such as compounds of formulae (A), (B), (C) and (D).

Those skilled in the art will appreciate that the process of the invention can be applied to essentially

any milbemycin or avermectin having a hydroxy (or protected hydroxy or oxo or oxime) group present at the C22 position. Furthermore, the compounds of formulae (I) and (III) can be further modified using techniques which are in themselves well known in the art and are described in, for example, Natural Products Reports 3 (2) [1986]87 et seq. and Macrolide Antibiotics [1984] Ch. 14, 553 et seq. Thus, a broad aspect of the invention provides any novel milbemycin or avermectin of partial formula (i) hereinbelow, as well as a process for its preparation which comprises the cyclization, such as acid-catalysed cyclisation, of a milbemycin or avermectin of partial formula (ii) hereinbelow:

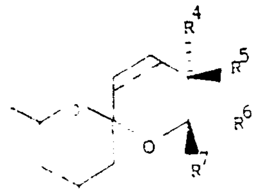

(i)

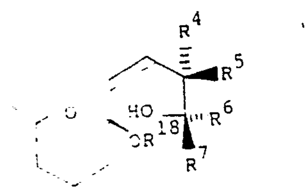

(ii)

wherein $R^4$ to $R^7$, $R^{18}$ and the dashed line have the values set out above. More especially, the substituents in the 6.6 spiroketal group (positions 22 to 25) are not the same as in any of the known compounds or derivable therefrom via known processes.

A further broad aspect of the invention provides a novel process for the preparation of milbemycins and avermectins of partial formula (iii):

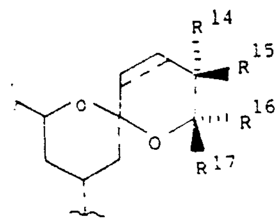

(iii)

wherein $R^{14}$ to $R^{17}$ are the same or different and each is selected from hydrogen and an organic radical; and the dashed line represents an optional double bond, which process comprises the cyclization, such as acid-catalysed cyclisation, of a corresponding milbemycin or avermectin of partial formula (iv):

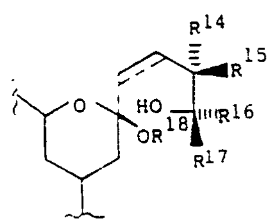

(iv)

wherein $R^{14}$ to $R^{18}$ and the dashed line have the values set out above.

Acetylenic precursors of structure $HC{\equiv}C.CR^4R^5CR^6R^7OP$ may be prepared by standard procedures, such as those shown schematically below:-

Route A

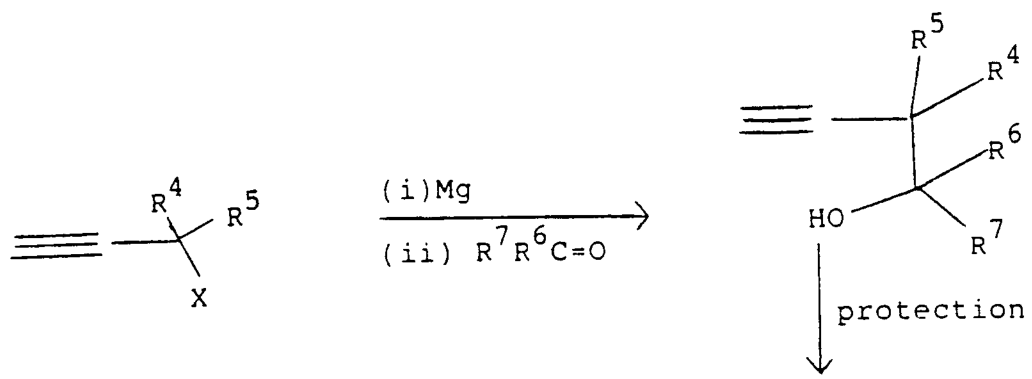


X = halogen eg Br

Where $R^4 \neq R^5$ or $R^6 \neq R^7$, this synthesis may give a mixture of isomers, which may either be separated, for example by silica gel chromatography, or used as a mixture in the subsequent reactions, and the resulting mixture of milbemycins separated by chromatography.

Route B

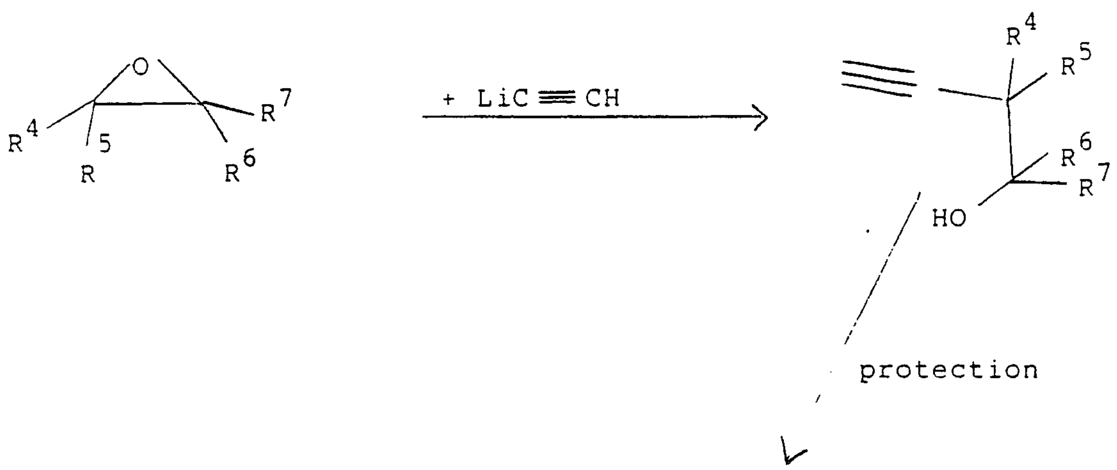


For a discussion of the regio- and stereoselectivity of this reaction, as well as the preparation of epoxides, reference can be made to a number of standard texts and reviews eg J. Gorzynski Smith, Synthesis (1984) 629-and Rao et al Tetrahedron 39, 2323, (1983).

The lithium acetylide may conveniently be used as its ethylenediamine complex.

Route C

This is a variation of route B in which a titanium acetylide is used in place of the lithium acetylide and this can be used to reverse the regioselectivity of the epoxide opening reaction. This technique is described by N. Krause and D. Seebach, Chem. Ber. 121, 1315-20 (1988).

It should be appreciated that it is possible to use chiral intermediates in Routes A to C to yield the acetylenic alcohol derivatives as single enantiomers.

The following Examples illustrate the present invention. VS 47709 is the compound of formula (III) wherein $R^1$ and $R^3$ are both hydrogen and $R^2$ is methoxy: and spiroketal-X is the compound of formula (I) wherein $R^1$ and $R^3$ to $R^7$ are hydrogen, $R^2$ is methoxy and the dashed line represents a single bond.

Example 1

Spiroketal-X

Washed and dried magnesium turnings were ground into small pieces. A mixture of 2-(3′-chloropropyl)-1,3-dioxolane (0.26 ml, 2 mmol), 1,2-dibromoethane (0.05 ml, 0.6 mmol) and dry THF (2 ml) was added to the ground magnesium (148 mg, 6 mmol) under $N_2$. The mixture was warmed and agitated to initiate reaction. The temperature was maintained at 40-45°C with stirring ($1\frac{3}{4}$ h). Dry THF (3 ml) was added and the mixture decanted from the excess magnesium and cooled to 0°C. Lactone (VS 47709) prepared as described in Examples 1 to 3 of EP-A-0 319 142 (USSN 265,509) (185 mg, 0.4 mmol) in dry THF (1 ml) was added and the mixture stirred at 0°C (30 min), treated with 1M aqueous ammonium chloride solution (20 ml) and extracted with ether (3 x 10 ml). The combined ethereal extracts were dried ($MgSO_4$) and evaporated. The product was purified by silica gel (5 g) column chromatography with hexane:ethyl acetate, 3:2 as eluant giving the hemiketal (151 mg, 65%) as a 5:2 mixture with its keto tautomer.

A mixture of hemiketal.ketone, prepared above, (150 mg, 0.26 mmol), THF (9 ml) and 2M hydrochloric acid (2 ml) was stirred at room temperature (6.5 h) until the starting material had been consumed by t.l.c. (hexane:ethyl acetate, 1:1). Sodium cyanoborohydride (100 mg, 1.56 mmol) was added. Water (ca 2 ml) was added to obtain a clear solution and the mixture stirred at room temperature (1 h), treated with water (20 ml) and extracted with ether (3 x 10 ml). The combined ethereal extracts were dried ($MgSO_4$) and evaporated.

The crude product (173 mg) was purified by silica gel (6 g) column chromatography with hexane:ethyl acetate, 4:1 as eluant giving the spiroketal X (56 mg, 42%) as a white solid; $\delta_C$ (270 MHz, $CDCl_3$,) 173.6, 142.2, 139.7, 137.0, 135.7, 123.5, 120.8, 119.3, 118.4, 97.2, 80.2, 77.3, 76.8, 68.4, 68.2, 67.6, 60.3, 57.7, 48.4, 45.5, 41.1, 36.3, 35.8, 35.1, 34.5, 24.9, 22.3, 19.8, 18.4 and 15.4, m/z (FAB) 537 ($MNa^+$, 100%).

Example 2

Spiroketal-X

Washed and dried magnesium turnings were ground into small pieces. A mixture of 1-chloro-4-(tetrahydropyran-2-yloxy)butane (388 mg, 2 mmol), 1,2-dibromoethane (0.05 ml, 0.6 mmol) and dry THF (2 ml) was added to the ground magnesium (146 mg, 6 mmol) under $N_2$. The mixture was warmed and agitated until reaction commenced. After the initial vigorous reaction had subsided the mixture was heated at approximately 45°C (1 h), cooled to room temperature and dry THF (3 ml) was added. The mixture was decanted from the excess magnesium and cooled to 0°C. Lactone (VS 47709) (90 mg, 0.2 mmol) in dry THF (1 ml) was added and the mixture stirred at 0°C (2 h). 2M Hydrochloric acid (10 ml) was added and the mixture stirred at room temperature (30 min), then extracted with ether (3 x 10 ml). The combined ethereal extracts were dried ($MgSO_4$) and evaporated. The product was identical with an authentic sample of spiroketal-X.

Example 3

(24R,25R)-22,23-Didehydro-24,25.dimethyl-X

To a solution of (3R*,4R*)-3-methyl-4-(tetrahydropyran)-2-yloxy)-1-pentyne (110 mg, 0.6 mmole) in THF (5 ml) at -78°C under nitrogen was added butyllithium (1.6M in hexane; 0.32 ml, 0.5 mmole) and the mixture was stirred at -78°C for $1\frac{1}{2}$ h. A solution of VS 47709 (90 mg, 0.2 mmole) in THF (1 ml) was added to the mixture which was then stirred at -78°C for 3 h. The reaction was quenched with dilute aqueous ammonium chloride (5 ml) and the mixture was then allowed to warm to 0°C. The aqueous phase was extracted with diethyl ether (2 x 20 ml) and the combined organic extracts were washed with water (10 ml), dried ($MgSO_4$) and evaporated to give a crude product (150 mg).

A solution of this crude product (150 mg) in ethyl acetate (20 ml) was hydrogenated over Lindlar catalyst (90 mg) for 16 h. The mixture was filtered through celite and evaporated to an oil.

To the unpurified product was added methanol (10 ml) and dilute hydrochloric acid (1M, 1 ml). The mixture was stirred for 1 h and evaporated to dryness to give the crude product. The residue was purified by preparative t.l.c. (silica plates eluted with ethyl acetate:hexane 30:70) to give the title compound (10 mg);

m z (FAB $Na^+$ NOBA) (relative intensity) 563 $[M + Na]^+$ (100%); $\delta_C$ ($CDCl_3$) 174.0, 142.5, 139.7, 137.0, 136.0. 135.5. 127.5, 123.5, 120.8, 119.4, 118.3, 96.2, 80.3, 77.4, 77.0, 68.6, 68.3, 68.2, 65.6, 57.8, 48.5, 45.6, 40.4, 36.3, 35.9, 34.9, 32.9, 22.3, 19.9, 17.9, 15.5, 12.1 ppm.

## Example 4

(24S.25R)-22.23-Didehydro-24,25-dimethyl-X and (24R,25S)-22,23-didehydro-24,25-dimethyl-X

In a similar manner to Example 3, (3R*,4S*)-3-methyl-4-(tetrahydropyran-2-yloxy)-1-pentyne (152 mg, 0.84 mmole) and VS 47709 (100 mg, 0.21 mmole) gave two products (24S,25R) and (24R,25S)-22,23-didehydro-24.25-dimethyl-X as a mixture. Purification by preparative HPLC gave (24R,25S)-22,23-didehydro-24.25-dimethyl-X (3.9 mg); m z (FAB $Na^+$ Noba) (relative intensity) 563 $[MNa^+]$ (100%); and (24S.25R)-22.23-didehydro-24,25-dimethyl-X (22.0 mg); m z (FAB $Na^+$ Noba) (relative intensity) 563 $[MNa^+]$ (100%); $\delta_C$ ($CDCl_3$) 173.8, 142.6, 142.4, 139.7, 137.1, 135.9, 135.4, 127.9, 123.5, 120.7, 119.4, 118.3, 95.7, 80.3, 77.4, 76.9, 69.7, 68.4, 68.3, 68.2, 57.8, 53.4, 48.5, 45.6, 40.3, 36.3, 36.1, 35.9, 34.8, 22.3, 19.9, 19.0, 16.6, 15.5 ppm.

## Example 5

(24S, 25S)-22.23-Didehydro-5-de-O-methyl-24-methyl-25-phenyl-X and (24R,25R)-22,23-didehydro-5-de-O-methyl-24-methyl-25-phenyl-X.

a) Addition of acetylide

A mixture of (3S*, 4S*)-3-methyl-4-phenyl-4-(t-butyldimethylsilyloxy)-1-butyne (475 mg, 1.73 mmol) and dry THF (10 ml) was cooled to -78°C under nitrogen. 1.6M-Butyllithium in hexane (1.0ml, 1.60 mmol) was added dropwise with stirring and the mixture stirred at -78°C (2h). A solution of 5-de-O-methyl VS 47709 prepared as described in Examples 1 to 3 of EP-A-0 319 142 (USSN 265,509) (190 mg, 0.43 mmol) in dry THF (1 ml) was rapidly added to the reaction mixture which was then stirred at -78°C (30 min). A solution of acetic acid (0.3 ml) in THF (5 ml) was cooled to -78°C and then rapidly added to the reaction mixture. After warming to R.T. 1M - ammonium chloride solution (50 ml) was added and the mixture extracted with ether (3 x 20 ml). The combined ethereal extracts were washed with saturated sodium bicarbonate solution (20 ml), dried ($MgSO_4$) and evaporated.

b) Deprotection and methoxy acetal formation

The residue was dissolved in methanol (10 ml). Toluene-4-sulphonic acid (ca 200 mg) was added and the mixture stirred at R.T. (17h), treated with water (50 ml) and extracted with ether (3 x 20 ml). The combined ethereal extracts were washed with saturated sodium bicarbonate solution (20 ml), dried ($MgSO_4$) and evaporated. The product was purified by silica gel column chromatography with light petroleum (b.p. 40-60°C)-ethyl acetate, 1:1 as eluant.

c) Reduction of alkyne to alkene and spirocyclisation

The product (76 mg) was dissolved in methanol (10 ml). 5% Palladium on barium sulphate catalyst (30 mg) and pyridine (0.1 ml) were added and the mixture stirred under a hydrogen atmosphere (1h). The catalyst was removed by filtration and washed with methanol.

The combined filtrate and washings were concentrated, treated with 1M-hydrochloric acid (50 ml) and then extracted with ether (3 x 20 ml). The combined ethereal extracts were dried ($MgSO_4$) and evaporated. The residue was purified by preparative t.l.c. (silica gel) with hexane-ethyl acetate, 1:1 as eluant affording a mixture of the title compounds (34 mg) which were separated by preparative h.p.l.c (methanol-water, 9:1).

(24R,25R-22,23-Didehydro-5-de-O-methyl-24-methyl-25-phenyl-X (9 mg) was eluted first, $^{13}$ C n.m.r. (270 MHz; $CDCl_3$) included 140.20, 128.28, 128.05, 127.91, 37.33, and 17.06, followed by (24S,25S)-22,23-Didehydro-5-de-O-methyl-24-methyl-25--phenyl--X, $^{13}C$ n.m.r. (270 MHz; $CDCl_3$) included 140.49, 128.23, 127.94, 127.65, 40.36, and 16.08.

## Example 6

### (24S, 25S)-5-De-O-methyl-24-methyl-25-phenyl-X.

A mixture of (24S, 25S)-22,23-didehydro-5-de-O-methyl-24-methyl-25-phenyl-X (89 mg), tris (triphenylphosphine) rhodium chloride (50 mg) and toluene (3 ml) was stirred under a hydrogen atmosphere (90 h). The reaction mixture was eluted through a short silica gel column with hexane-ethyl acetate, 3:1. The product was purified by preparative h.p.l.c. (methanol-water, 9:1) giving (24S, 25S)-5-de-O-methyl-24-methyl-25-phenyl-X (14 mg). $^{13}$ C n.m.r. (270 MHz; $CDCl_3$) included 141.22, 128.08, 127.68, 127.63, 41.18, and 17.80.

The compounds of formula (I) given in the following Table, wherein $R^1 = R^3 = H$ and the dashed line represents a double bond, were prepared using the method of Example 5:

| Ex.No. | $R^2$ | $R^7$ | $R^4$ | $R^6$ | $R^5$ |
|---|---|---|---|---|---|
| 7 | $OCH_3$ | H | H | 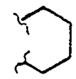 | |
| 8 | $OCH_3$ | 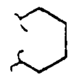 | | H | H |
| 9 | $OCH_3$ | H | H | 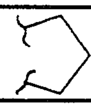 | |
| 10 | $OCH_3$ | 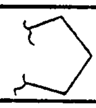 | | H | H |
| 11 | $OCH_3$ | $C_6H_5$ | H | H | H |
| 12 | $OCH_3$ | H | H | $C_6H_5$ | H |
| 13 | $OCH_3$ | H | H | n-$C_6H_{13}$ | H |
| 14 | $OCH_3$ | n-$C_6H_{13}$ | H | H | H |
| 15 | $OCH_3$ | H | H | $C_6H_5$ | $CH_3$ |
| 16 | $OCH_3$ | $C_6H_5$ | $CH_3$ | H | H |
| 17 | $OCH_3$ | n-$C_5H_{11}$ | n-$C_5H_{11}$ | H | H |
| 18 | $OCH_3$ | H | H | n-$C_5H_{11}$ | n-$C_5H_{11}$ |
| 19 | $OCH_3$ | H | H | $C_2H_5$ | $CH_3$ |
| 20 | $OCH_3$ | H | H | $CH_3$ | $C_2H_5$ |
| 21 | $OCH_3$ | $C_2H_5$ | $CH_3$ | H | H |
| 22 | $OCH_3$ | $CH_3$ | $C_2H_5$ | H | H |
| 23 | $OCH_3$ | 4-Cl.$C_6H_4$ | H | H | H |
| 24 | $OCH_3$ | H | H | 4-Cl.$C_6H_4$ | H |
| 25 | OH | H | H | $C_6H_5$ | H |
| 26 | OH | $C_6H_5$ | H | H | H |
| 27 | $OCH_3$ | H | H | Cyclo-$C_6H_{11}$ | H |
| 28 | $OCH_3$ | Cyclo-$C_6H_{11}$ | H | H | H |
| 29 | $OCH_3$ | H | H | $CH_3$ | $C_6H_5$ |
| 30 | $OCH_3$ | $CH_3$ | $C_6H_5$ | H | H |

| Ex.No. | $R^2$ | $R^7$ | $R^4$ | $R^6$ | $R^5$ |
|---|---|---|---|---|---|
| 31 | $OCH_3$ | 2-Furyl | H | H | H |
| 32 | $OH_3$ | H | H | 2-Furyl | H |
| 33 | $OCH_3$ | H | H | n-$C_5H_{11}$ | $CH_3$ |
| 34 | $OCH_3$ | n $C_5H_{11}$ | $CH_3$ | H | H |
| 35 | OH | 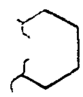 | | H | H |
| 36 | $OCH_3$ | H | H | t-$C_4H_9$ | H |
| 37 | $OCH_3$ | t-$C_4H_9$ | H | H | H |
| 38 | $OCH_3$ | 4-$CH_3O.C_6H_4$ | H | H | H |
| 39 | $OCH_3$ | H | H | 4-$CH_3O$ $C_6H_4$ | H |
| 40 | $OCH_3$ | H | H | $CH_3$ | n-$C_5H_{11}$ |
| 41 | $OCH_3$ | $CH_3$ | n-$C_5H_{11}$ | H | H |
| 42 | $OCH_3$ | 3-Cl.$C_6H_4$ | H | H | H |
| 43 | $OCH_3$ | H | H | 3-Cl.$C_6H_4$ | H |
| 44 | $OCH_3$ | 3-Furyl | H | H | H |
| 45 | $OCH_3$ | H | H | 3-Furyl | H |
| 46 | $OCH_3$ | 3-$CH_3.C_6H_4$ | H | H | H |
| 47 | $OCH_3$ | H | H | 3-$CH_3.C_6H_4$ | H |
| 48 | $OCH_3$ | H | H | 3-$CH_3.C_6H_4$ | H |
| 49 | $OCH_3$ | 4-$CH_3.C_6H_4$ | H | H | H |
| 50 | $OCH_3$ | H | H | 4-$CH_3.C_6H_4$ | H |
| 51 | $OCH_3$ | 4-F.$C_6H_4$ | H | H | H |
| 52 | $OCH_3$ | H | H | 4-F.$C_6H_4$ | H |

| Ex.No | $R^2$ | $R^7$ | $R^4$ | $R^6$ | $R^5$ |
|---|---|---|---|---|---|
| 53 | OH | H | H | $C_6H_5$ | $CH_3$ |
| 54 | OH | $C_6H_5$ | $CH_3$ | H | H |
| 55 | $OCH_3$ | cyclo-$C_5H_9$ | H | H | H |
| 56 | $OCH_3$ | H | H | cyclo-$C_5H_9$ | H |
| 57 | $OCH_3$ | H | H | cyclo-$C_7H_{13}$ | H |
| 58 | $OCH_3$ | cyclo-$C_7H_{13}$ | H | H | H |
| 59 | $OCH_3$ | H | H | 2-Thienyl | H |
| 60 | $OCH_3$ | 2-Thienyl | H | H | H |
| 61 | $OCH_3$ | 2-$CH_3O.C_6H_4$ | H | H | H |
| 62 | $OCH_3$ | H | H | 2-$CH_3O.C_6H_4$ | H |
| 63 | OH | cyclo-$C_6H_{11}$ | H | H | H |
| 64 | OH | H | H | t-$C_4H_9$ | H |
| 65 | OH | t-$C_4H_9$ | H | H | H |
| 66 | OH | H | H | n-$C_6H_{13}$ | H |
| 67 | OH | n-$C_6H_{13}$ | H | H | H |
| 68 | OH | 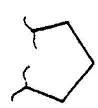 | | H | H |
| 69 | OH | H | H | cyclo-$C_6H_{11}$ | $CH_3$ |
| 70 | OH | Cyclo.$C_6H_{11}$ | $CH_3$ | H | H |
| 71 | OH | $CH_3$ | n-$C_5H_{11}$ | H | H |
| 72 | OH | H | H | 4-$CH_3.C_6H_4$ | H |
| 73 | OH | 4-$CH_3.C_6H_4$ | H | H | H |
| 74 | OH | H | H | t-$C_4H_9$ | $CH_3$ |
| 75 | OH | t-$C_4H_9$ | $CH_3$ | H | H |
| 76 | OH | n-$C_5H_{11}$ | $CH_3$ | H | H |

| Ex.No | $R^2$ | $R^7$ | $R^4$ | $R^6$ | $R^5$ |
|---|---|---|---|---|---|
| 77 | OH | H | H | $\underline{n}$-$C_5H_{11}$ | $CH_3$ |
| 78 | $OCH_3$ | H | H | H | $C_6H_5$ |
| 79 | $OCH_3$ | H | $C_6H_5$ | H | H |
| 80 | OH | 4-F.$C_6H_4$ | H | H | H |
| 81 | OH | H | H | 4-F.$C_6H_4$ | H |
| 82 | OH | H | H | $C_2H_5$ | $C_6H_5$ |
| 83 | OH | $C_2H_5$ | $C_6H_5$ | H | H |
| 84 | OH | H | H | $C_6H_5$ | $C_2H_5$ |
| 85 | OH | $C_6H_5$ | $C_2H_5$ | H | H |
| 86 | OH | H | H | i-$C_3H_7$ | $CH_3$ |
| 87 | $OCH_3$ | H | H | i-$C_3H_7$ | $CH_3$ |
| 88 | $OCH_3$ | $\underline{i}$-$C_3H_7$ | $CH_3$ | H | H |
| 89 | OH | $\underline{i}$-$C_3H_7$ | $CH_3$ | H | H |
| 90 | $OCH_3$ | 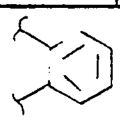 | | - | - |
| 91 | OH | H | H | 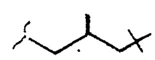 | H |
| 92 | OH | 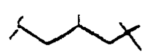 | H | H | H |
| 93 | OH | H | $\underline{n}$-$C_4H_9$ | -$C_6H_5$ | H |
| 94 | OH | -$C_6H_5$ | H | H | $\underline{n}$-$C_4H_9$ |
| 95 | OH | H | H | -$C_6H_5$ | $\underline{n}$-$C_4H_9$ |
| 96 | OH | -$C_6H_5$ | $\underline{n}$-$C_4H_9$ | H | H |
| 97 | OH | 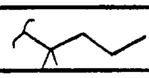 | $CH_3$ | H | H |
| 98 | OH | H | H | 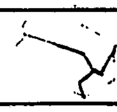 | H |
| 99 | OH | 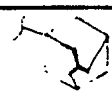 | H | H | H |
| 100 | OH | H | H | -$C_6H_5$ | $\underline{n}$-$C_3H_7$ |

| Ex.No | $R^2$ | $R^7$ | $R^4$ | $R^6$ | $R^5$ |
|---|---|---|---|---|---|
| 101 | OH | $-C_6H_5$ | n-$C_3H_7$ | H | H |
| 102 | OH | 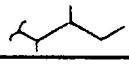 | H | H | H |

The compounds of formula (I) given in the following Table, wherein $R^1 = R^3 = H$ and the dashed line represents a single bond, were prepared using the method of Example 6:

| Ex.No | $R^2$ | $R^7$ | $R^4$ | $R^6$ | $R^5$ |
|---|---|---|---|---|---|
| 103 | $OCH_3$ | H | H | $C_6H_5$ | H |
| 104 | $OCH_3$ | 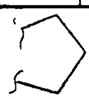 | | H | H |
| 105 | OH | $C_6H_5$ | $CH_3$ | H | H |

Preparation 1

(3R*,4R*)-3-Methyl-1-pentyn-4-ol

Prepared as described in the literature from trans-2,3-epoxybutane and lithium acetylide, ethylenediamine complex in dimethylsulphoxide (J Am Chem Soc (1970) 101, 5367).

Preparation 2

(3R*,4R*)-3-Methyl-4-(tetrahydropyran-2-yloxy)-1-pentyne

To dihydropyran (1.3 g) cooled in an ice bath was added (3R*,4R*)-3-methyl-1-pentyn-4-ol (1.3 g) followed by concentrated hydrochloric acid (1 drop) and the mixture was stirred for 16 h at room temperature. Potassium carbonate (100 mg) was added and the mixture stirred for 15 minutes. Diethyl ether (10 ml) was added and the mixture was filtered before evaporation to an oil. The residue was distilled to give the title compound (1.2 g) (b.p. 130-5°C at 10 mm Hg).

Preparation 3

(3R*,4S*)-3-Methyl-1-pentyn-4-ol

To a suspension of lithium acetylide ethylenediamine complex (4 g, 44 mmole) in dimethyl sulphoxide (10 ml) was added cis-2,3-epoxybutane (2.9 g, 40 mmole) and the mixture was stirred under nitrogen for 10 days at room temperature. The reaction was quenched with water (25 ml) and extracted with diethyl ether (3

x 50 ml). The combined ethereal extracts were dried ($MgSO_4$) and evaporated to give an oil. The residue was distilled to give the title compound as a colourless oil (1.2 g) (b.p. 35-45°C at 30 mm Hg).

Preparation 4

(3R*,4S*)-3-Methyl-4-(tetrahydropyran-2-yloxy)-1-pentyne

In a similar manner to Preparation 2 (3R*,4S*)-3-methyl-1-pentyn-4-ol gave the title compound as a colourless oil (1.2 g) (b.p. 115-20°C at 40 mm Hg).

Preparation 5

3-Bromo-1-heptyne

A solution of phosphorous tribromide (9.6g, 0.036 mole) in ether was added to a stirred mixture of 1-heptyn-3-ol (10g, 0.195 mole) and pyridine (0.78g, 0.0098 mole) in ether (50ml) over a period of 1 hour, the internal temperature being kept at -70°C. After the mixture had been stirred at this temperature for 1 hour the mixture was then allowed to warm to O°C and water (100ml) carefully added. The ether layer was separated from the aqueous layer, washed with water, sodium bicarbonate solution and brine before drying ($MgSO_4$). Evaporation gave the crude product (7.5g) which was purified by distillation to give the title compound (3.3g) b.p. 76° 40mm.

Preparation 6

3-Butyl-4-phenyl-1-butyn-4-ol.

A solution of 3-bromo-1-heptyne (3.3g, 0.019 mole) in ether (5ml) was slowly added to a stirred mixture of magnesium turnings (450 mg, 0.019 mole) and mercuric chloride (15 mg) in ether (10 ml). Once the reaction had been initiated by gentle heating the rate of addition of the bromide was set to maintain refluxing of the ether. After 1 hour the mixture was cooled to O°C and benzaldehyde (2.0g, 0.019 mole) in ether (3 ml) was slowly added. The mixture was stirred for 2 hour at 0°C before addition of saturated aqueous ammonium chloride solution. The ether layer was separated, washed with water, dried ($MgSO_4$) and evaporated to give the crude product (3.5g). Purification by column chromatography using silica gel gradient eluted with hexane to ether/hexane (15/85) afforded (3R*,4S*)-3-butyl-4-phenyl-1-butyn-4-ol (900 mg) t.l.c. $SiO_2$, ether/hexane (20/80) Rf 0.35 and (3R*,4R*)-3-butyl-4-phenyl-1-butyn-4-ol (1g). t.l.c. $SiO_2$, ether hexane (20/80) Rf 0.3.

Preparation 7

3-phenyl-1-butyn 4-ol

2-phenyl-4-trimethylsilyl-3-butyn-1-ol was prepared as described in the literature:-
D Seebach et al Chem. Ber. 121 1315-20 (1988).
Cleavage of the trimethylsilyl protecting group with methanolic potassium hydroxide solution gave the title compound
In addition, the compounds of formula $HC\equiv C.CHR^{12}CHR^{13}OP$ given in the following table were prepared by procedures analogous to those described in preparations 3 to 7. Protection of the alcohols was achieved by standard methods such as those described in "Protective groups in organic synthesis"by Theodora W. Greene, publ. John Wiley and Sons.

| Stereochemistry | $R^{12}$ | $R^{13}$ | P | Route of Preparation |
|---|---|---|---|---|
| - | H | $C_6H_5$ | TBDMS | B |
| - | H | n-$C_6H_{13}$ | TBDMS | B |
| - | H | 4-Cl.$C_6H_4$ | TBDMS | B |
| - | H | Cyclo-$C_6H_{11}$ | TBDMS | B |
| - | H | 3-Cl.$C_6H_4$ | TBDMS | B |
| - | H | 4-$CH_3O$.$C_6H_4$ | TBDMS | A |
| - | H | 4-$CH_3$.$C_6H_4$ | TBDMS | A |
| - | H | 4-F.$C_6H_4$ | TBDMS | A |
| - | H | 2-$CH_3O$.$C_6H_4$ | TBDMS | A |
| - | H | Cyclo-$C_7H_{13}$ | TBDMS | A |
| - | H | 2-thienyl | TBDMS | B |
| - | H | Cyclo-$C_5H_{11}$ | TBDMS | A |
| - | H | 3-Furyl | TBDMS | A |
| - | H | 2-Furyl | TBDMS | B |
| - | H | $CH_3$ | THP | B |
| - | H | tert-$C_4H_9$ | TBDMS | B |

| Stereochemistry | $R^{12}$ | $R^{13}$ | P | Route of Preparation |
|---|---|---|---|---|
| Trans | $n$-$C_5H_{11}$ | $n$-$C_5H_{11}$ | TBDMS | B |
| cis | $n$-$C_5H_{11}$ | $n$-$C_5H_{11}$ | TBDMS | B |
| Trans | $CH_3$ | Cyclo-$C_6H_{11}$ | TBDMS | A |
| Trans | $CH_3$ | tert $C_4H_9$ | TBDMS | A |
| Trans | $CH_3$ | tert $C_4H_9$ | TMS | A |
| Trans | $n$-$C_4H_9$ | $C_6H_5$ | TBDMS | A |
| cis | $n$-$C_4H_9$ | $C_6H_5$ | TBDMS | A |
| Trans | $C_6H_5$ | $C_2H_5$ | TBDMS | B |
| Trans | $C_2H_5$ | $C_6H_5$ | TBDMS | C |
| Trans | $CH_3$ | $CH_3$ | THP | B |
| cis | $CH_3$ | $CH_3$ | THP | B |
| Trans | $CH_3$ | $C_6H_5$ | TBDMS | B |
| Trans | $CH_3$ | $C_6H_5$ | TBDMS | A |
| cis | $CH_3$ | $C_6H_5$ | TBDMS | A |
| Trans (s,s) | $CH_3$ | $C_6H_5$ | TBDMS | B |

| Stereochemistry | $R^{12}$ | $R^{13}$ | P | Route of Preparation |
|---|---|---|---|---|
| Trans | $CH_3$ | $C_2H_5$ | TBDMS | B |
| Trans | $C_2H_5$ | $CH_3$ | TBDMS | B |
| Trans | $CH_3$ | $C_5H_{11}$ | TBDMS | B |
| Trans | $C_5H_{11}$ | $CH_3$ | TBDMS | B |
| cis | $C_6H_5$ | $CH_3$ | TBDMS | A |
| Trans | $C_6H_5$ | $CH_3$ | TBDMS | A |
| - | $C_6H_5$ | H | TBDMS | C |
| Trans | $CH_3$ | i-$C_3H_7$ | TBDMS | A |
| Trans | $CH_3$ | t-$C_4H_9$ | TBDMS | A |
| Trans | 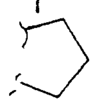 | | TBDMS | B |
| Trans | 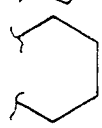 | | THP | B |
| | 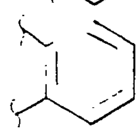 | | TBDMS | Literature compound |
| -- | H | 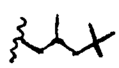 | TBDMS | A |
| cis | n-$C_4H_9$ | $C_6H_5$ | TBDMS | A |
| Trans | n-$C_4H_9$ | $C_6H_5$ | TBDMS | A |
| Trans | $CH_3$ | 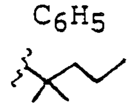 | TMS | A |
| -- | H | 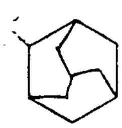 | TBDMS | A |
| Trans | n-$C_3H_5$ | $C_6H_5$ | TBDMS | A |
| -- | H | 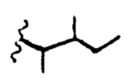 | TBDMS | A |

Temperatures given in the Examples are in Celsius unless otherwise indicated.

## Claims

1. A compound of formula (I)

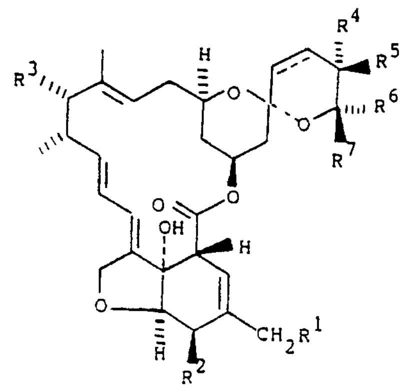

(I)

wherein R' is hydrogen or optionally protected hydroxy; $R^2$ is alkoxy, optionally protected hydroxy, oxo or optionally O-substituted oximino; $R^3$ is hydrogen, optionally protected hydroxy,or a group 4'-(α-L-oleandrosyl)-α-L-oleandrosyloxy or α-L-oleandrosyloxy wherein the terminal hydroxy group is optionally protected; the dashed line represents an optional double bond; the $R^4$, $R^5$, $R^6$ and $R^7$ are the same or different and each is hydrogen or an organic radical; with the proviso that the compound of formula (I) is not a compound disclosed by GB-A-1,573,955, GB-A-1,390,336, GB-A-2 176182, EP-A-0 212 867, EP-A-0 237 339, EP-A-0 241 146, EP-A-0 214 731, EP-A-0 194 125, EP-A-0 246 739, EP-A-0 316 124 or EP-A-0 317 148.

2. A process for the preparation of a compound of formula (I) as defined in claim 1, which process comprises the cyclisation of a compound of formula (II):

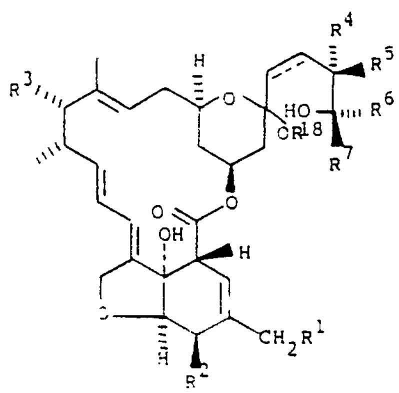

(II)

wherein R' to $R^7$ and the dashed line have the values set out in claim 1 and $R^{18}$ is hydrogen or lower alkyl; and/or, optionally the conversion of a compound of formula (I) wherein the dashed line represents a double bond to a compound of formula (I) wherein the dashed line represents a single bond.

3. A milbemycin or avermectin of partial formula (i):

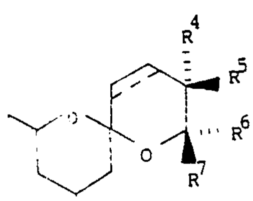

(i)

wherein $R^4$ to $R^7$ and the dashed line have the values set out in claim 1, with the proviso that (a) when $R^4$ is methyl and $R^5$ and $R^6$ are hydrogen, $R^7$ does not have any of the values set out in Table I hereinbefore, (b) when the double bond is absent, $R^4$ is methyl and $R^5$ and $R^6$ are hydrogen, $R^7$ does not have any of the values set out in Table II hereinabove, (c) when the double bond is absent, $R^4$ is hydroxymethyl and $R^5$

and $R^6$ are hydrogen, $R^7$ is not <u>sec</u> butyl, and (d) the compound of formula (i) is not disclosed in EP-A-0 317 148 or EP-A-0 316 124.

4. A process for the preparation of a compound of formula (i) as defined in claim 3, which process comprises the cyclisation of a compound of partial formula (ii):

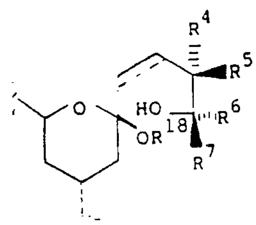

(ii)

wherein $R^4$ to $R^7$ and the dashed line have the values set out in claim 3 and $R^{'8}$ is hydrogen or lower alkyl, and, optionally, the conversion of a compound of formula (i) wherein the dashed line represents a double bond to a compound of formula (i) wherein the dashed line represents a single bond.

5. A process for the preparation of a milbemycin or avermectin of partial formula (iii):

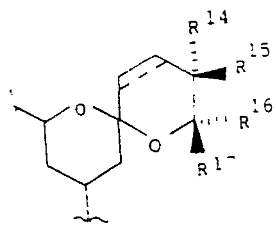

(iii)

wherein $R^{'4}$ to $R^{'7}$ are the same or different and.each is selected from hydrogen and an organic radical; and the dashed line represents an optional double bond, which process comprises the cyclization of a corresponding milbemycin or avermectin of partial formula (iv):

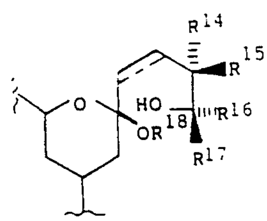

(iv)

wherein $R^{'8}$ is hydrogen or lower alkyl, and, optionally, the conversion of a compound of formula (iii) wherein the dashed line represents a double bond to a compound of formula (iii) wherein the dashed line represents a single bond.

6. A process according to claim 5, wherein the compound of partial formula (iii) is a compound of formula (iiia):

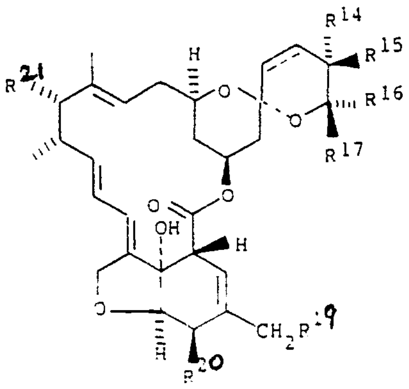

(iiia)

wherein $R'^4$ to $R'^7$ and the dashed line have the values set out in claim 5, and $R'^9$ to $R^{2'}$ have the values set out in claim 1 with respect to R' to $R^3$.

7. A milbemycin compound as defined in any one of Examples 1 to 105 hereinbefore.

8. A compound according to any one of claims 1, 3 and 7, for use in the treatment or prophylaxis of endo-and ectoparasitic infestations of the human or animal body.

9. A method of eradicating arthropod or nematode infestations, which method comprises applying a compound according to any one of claims 1, 3 and 7, or a derivative thereof to the arthropods or nematodes or to their environment.

10. A pesticidal composition comprising a compound according to any one of claims 1, 3 and 7 or a derivative thereof together with an inert carrier or excipient.

11. A pharmaceutical or veterinary composition comprising a compound according to any one of claims 1, 3 and 7, or a pharmaceutically or veterinarily acceptable derivative thereof together with a pharmaceutically or veterinarily acceptable carrier or excipient.

Claims for the following Contracting State: ES

A process for the preparation of a compound of formula (I):

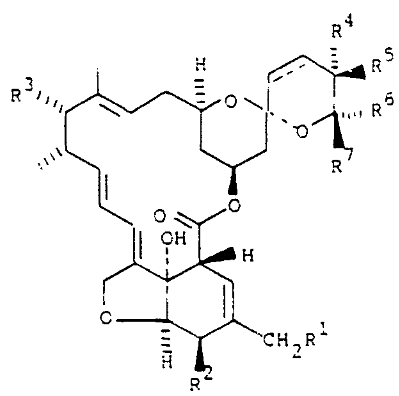

(I)

wherein $R^1$ is hydrogen or optionally protected hydroxy; $R^2$ is alkoxy, optionally protected hydroxy, oxo or optionally O-substituted oximino; $R^3$ is hydrogen, optionally protected hydroxy, or a group 4'-(α-L-oleandrosyl)-α-L-oleandrosyloxy or α-L-oleandrosyloxy wherein the terminal hydroxy group is optionally protected; the dashed line represents an optional double bond; and $R^4$, $R^5$, $R^6$ and $R^7$ are the same or different and each is hydrogen or an organic radical; with the proviso that the compound of formula (I) is not a compound disclosed by GB-A-1,573,955, GB-A-1,390,336, GB-A-2 176 182, EP-A-0 212 867, EP-A-0 237 339, EP-A-0 241 146, EP-A-0 214 731, EP-A-0 194 125, EP-A-0 246 739, EP-A-0 316 124 or EP-A-0 317 148, which process comprises the cyclisation of a compound of formula (II):

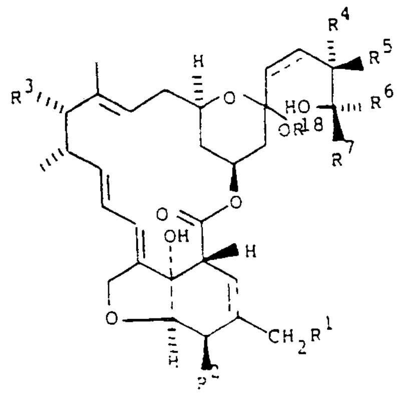

(II)

wherein $R^1$ to $R^7$ and the dashed line have the values set out above and $R^{18}$ is hydrogen or lower alkyl; and or. optionally. the conversion of a compound of formula (I) wherein the dashed line represents a double bond to a compound of formula (I) wherein the dashed line represents a single bond.

2. A process for the preparation of a compound of formula (i):

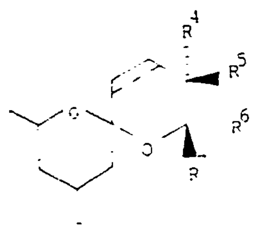

(i)

wherein $R^4$ to $R^7$ and the dashed line have the values set out in claim 1, with the proviso that (a) when $R^4$ is methyl and $R^5$ and $R^6$ are hydrogen, $R^7$ does not have any of the values set out in Table I hereinabove, (b) when the double bond is absent, $R^4$ is methyl and $R^5$ and $R^6$ are hydrogen. $R^7$ does not have any of the values set out in Table II hereinabove, (c) when the double bond is absent, $R^4$ is hydroxymethyl and $R^5$ and $R^6$ are hydrogen, $R^7$ is not sec butyl, and (d) the compound of formula (i) is not disclosed in EP-A-0 317 148 or EP-A-0 316 124, which process comprises the cyclisation of a compound of partial formula (ii):

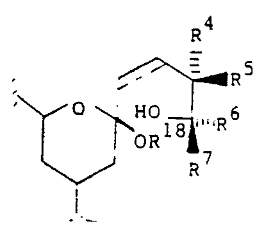

(ii)

wherein $R^4$ to $R^7$ and the dashed line have the values set out above and $R^{18}$ is hydrogen or lower alkyl, and, optionally, the conversion of a compound of formula (i) wherein the dashed line represents a double bond to a compound of formula (i) wherein the dashed line represents a single bond.

3. A process for the preparation of a milbemycin or avermectin of partial formula (iii):

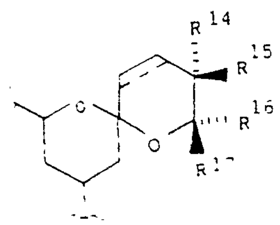

(iii)

wherein $R^{14}$ to $R^{17}$ are the same or different and each is selected from hydrogen and an organic radical; and the dashed line represents an optional double bond, which process comprises the cyclization of a corresponding milbemycin or avermectin of partial formula (iv):

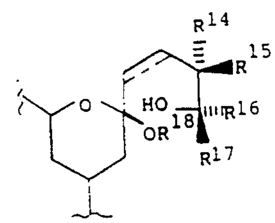

(iv)

wherein $R^{18}$ is hydrogen or lower alkyl, and, optionally, the conversion of a compound of formula (iii) wherein the dashed line represents a double bond to a compound of formula (iii) wherein the dashed line represents a single bond.

4. A process according to claim 3, wherein the compound of partial formula (iii) is a compound of formula (iiia):

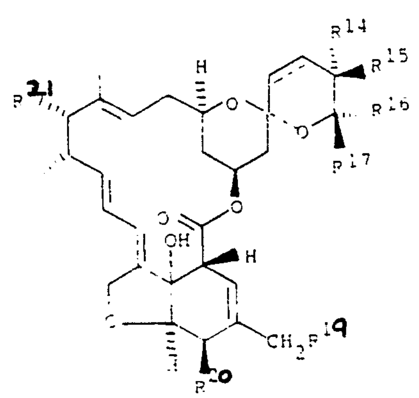

(iiia)

wherein $R^{14}$ to $R^{17}$ and the dashed line have the values set out in claims 3, and $R^{19}$ to $R^{21}$ have the values set out in claim 1 with respect to $R^1$ to $R^3$.

5. A process according to any preceding claim, for the preparation of a milbemycin compound as defined in any one of Examples 1 to 105 hereinbefore.

6. Use of a compound of formula (i) as defined in claim 2 for the manufacture of a medicament for use in the treatment or prophylaxis of endo- and ectoparasitic infestations of the human or animal body.

7. A method of eradicating arthropod or nematode infestations, which method comprises applying an effective amount of a compound of formula (i) or a derivative thereof, to the arthropods or nematodes or to their environment.

8. A process for the preparation of a pesticidal composition comprising mixing a compound formula (i) with an inert carrier or excipient.

9. A process for the preparation of a pharmaceutical or veterinary composition comprising mixing a compound formula (i) or a pharmaceutically or veterinarily acceptable derivative thereof with a pharmaceutically or veterinarily acceptable carrier or excipient.